# FASCICULE DE BREVET EUROPEEN

(11) **EP 3 194 354 B1**
(45) Date de publication et mention de la délivrance du brevet: **14.07.2021**
(21) Numéro de dépôt: 15770478.4
(22) Date de dépôt: 15.09.2015
(51) Int. Cl.: C07C 39/08, B65D 81/24, B01J 4/00, B01J 19/00, B65D 81/34

(54) **PROCEDE DE DECHARGEMENT A L'ETAT LIQUIDE D'UN COMPOSE OXYDABLE**
VERFAHREN ZUM ABGABE EINER OXIDIERBAREN VERBINDUNG IN FLÜSSIGEM ZUSTAND
METHOD FOR DISCHARGING AN OXIDISABLE COMPOUND IN THE LIQUID STATE

(30) Priorité: 18.09.2014 FR 1458828
(43) Date de publication de la demande: 26.07.2017
(73) Titulaire: Rhodia Operations, 93300 Aubervilliers (FR)
(72) Inventeur: FISCHER, Lars, F-38200 Vienne (DE); CAVEZZAN, Jacques, F-69100 Villeurbanne (FR); THOME, Jean-François, F-69390 Vourles (FR)
(74) Mandataire: Jacques, Marie-Caroline Anne
(86) Numéro de dépôt international: PCT/EP2015/071062
(87) Numéro de publication internationale: WO 2016/041943

(56) Documents cités:
- EP-A1- 0 364 850
- WO-A1-2007/024023
- US-B1- 6 371 756
- US-B1- 6 412 670
- US-B1- 6 517 057
- CHATFIELD ED - CHATFIELD H W: "WAXES", 1 January 1953 (1953-01-01), VARNISH CONSTITUENTS, LONDON, LEONARD HILL LIMITED, GB, PAGE(S) 625 - 647, XP000673297,

## Description

### DOMAINE TECHNIQUE DE L'INVENTION

La présente invention concerne la manutention de produits oxydables à l'état solide à température ambiante. Plus spécifiquement, cette invention concerne l'étape de déchargement d'un tel produit à l'état liquide, mais aussi les étapes de chargement, de stockage et de transport.

### ETAT DE LA TECHNIQUE

Les inhibiteurs de polymérisation sont des composés chimiques capables d'empêcher les réactions de polymérisation entre monomères. Ce sont donc des composés très utilisés industriellement, qui sont ajoutés dans les containers de monomères pour faciliter et sécuriser leur stockage, leur transport ou leur distillation. Parmi ces inhibiteurs, le para-méthoxyphénol (PMP) est un composé bien connu, qui se présente généralement sous la forme d'un solide, sa température de fusion étant d'environ 55°C. Or, au niveau industriel, une composition sous forme liquide peut être préférée car celle-ci est plus facile à pomper et à mélanger par rapport à une forme solide. Une difficulté technique réside cependant dans le fait que le PMP est sensible à l'oxydation. En effet, s'il est laissé dans un milieu oxydant, par exemple à l'air, le PMP sous forme liquide réagit et les sous-produits formés peuvent être colorés. Or, la coloration du composé est un problème important car il peut entrainer également la coloration de la composition polymère résultant de la polymérisation du monomère, ce qui peut être hautement indésirable en fonction des applications finales de la composition polymère.

Dans le brevet US 6 517 057, un système de distribution d'un composé inhibiteur de polymérisation dans une cuve de matière polymérisable est décrit. Ce système, qui présente l'avantage d'être fiable et simple, n'est adapté cependant qu'à la distribution d'un inhibiteur sous forme liquide, tel qu'une solution de phénothiazine (PTZ) liquide à température ambiante. Ce dispositif n'est pas adapté au cas où le composé inhibiteur de polymérisation est sous forme solide. L'utilisation d'inhibiteur en solution n'est pas souhaitable. D'une part, des traces de solvant peuvent se retrouver de façon non désirable dans les solutions de monomères. D'autre part, le transport de composé en solution représente un surcout énergétique et financier non souhaitable dans un contexte de développement durable.

La demande de brevet EP 0 364 850 décrit un dispositif pour le stockage dans un container d'un matériau sensible à l'air ou à l'humidité et un procédé de déchargement dudit matériau. Ce document décrit notamment l'utilisation de plaques chauffantes pour liquéfier le matériau sensible à l'air ou à l'humidité. Cependant, aucune indication n'est donnée sur la manière et la température de chauffage.

La demande internationale WO 2007/024023 décrit quant à elle un procédé de production d'un matériau en résine polyoxyde d'alkylène qui comprend une étape de chauffage de la surface supérieure de la résine. La seule indication concernant la manière de procéder à ce chauffage est que la température de chauffage ne doit pas excéder 300°C, ce qui, pour une résine dont le point de fusion est compris entre 0°C et 60°C, constitue une limite très élevée. US-A-6 371 756 décrit un procédé de réchauffement de cire solide.

C'est dans ce contexte que les inventeurs ont cherché un procédé permettant de décharger, à l'état liquide, un composé solide à température ambiante, sans rencontrer les problèmes de dégradation et de coloration du composé. Plus généralement, un des objectifs est de proposer un procédé de déchargement d'un composé oxydable ayant un point de fusion supérieur ou égal à 15°C.

On souhaite en outre que le déchargement soit facile à mettre en œuvre, qu'il ne requiert pas de moyens couteux et que la quantité de produit perdu lors du déchargement soit aussi faible que possible, de préférence négligeable. Avantageusement, on souhaite aussi éviter tout risque de bouchage de l'installation de vidange depuis le conteneur jusqu'au bac de stockage ou d'utilisation du produit.

### DESCRIPTION DE L'INVENTION

L'invention a pour objet un procédé de déchargement d'un container contenant un composé oxydable ayant un point de fusion T_{f} supérieur à 15°C, comprenant les étapes consistant à :
a) chauffer le container à l'aide d'un moyen de chauffage jusqu'à ce que le composé oxydable soit liquide, la température du moyen de chauffage restant inférieure ou égale à T_{f} + 105°C ;
b) décharger le composé oxydable à l'état liquide.

L'invention a également pour objet un procédé de manutention d'un composé oxydable ayant un point de fusion T_{f} supérieur à 15°C comprenant les étapes de chargement d'un container avec un composé oxydable, de stockage et/ou de transport du container chargé, de déchargement du container, et éventuellement de retour du container après déchargement, caractérisé en ce que l'étape de déchargement est mise en œuvre comme décrit ci-dessus.

Enfin, un dispositif comprenant (A) un container utilisable dans les procédés décrits ci-dessus et (B) des instructions pour procéder au déchargement dudit container est également proposé ici.

Dans l'exposé qui suit, l'expression « compris entre ... et ... » doit être comprise comme incluant les bornes citées.

L'objet de la présente invention est lié à la manutention d'un composé oxydable ayant un point de fusion T_{f} supérieur à 15°C

Dans l'exposé qui suit, l'expression « composé oxydable » désigne notamment un composé chimique qui est susceptible de se transformer lorsqu'il est mis en contact avec un milieu oxydant, en particulier un milieu contenant de l'oxygène. Le composé oxydable selon l'invention peut être un composé qui subit une dégradation non désirée lorsqu'il est placé à l'air. Cette dégradation peut notamment avoir pour conséquence un changement de couleur ou la formation de composés lourds qui peuvent occasionner une perte de produit ou un dépôt sur les parois des contenants. Il peut s'agir d'un composé oxydable à température ambiante ou bien à une température supérieure à la température ambiante, par exemple à une température à laquelle le composé est soumis lors de son transport, son stockage ou son utilisation. Les composés réputés pour leur grande stabilité à l'air, tels que les cires de bougies, ne sont donc pas concernés par la présente invention.

Le composé oxydable selon l'invention a un point de fusion (également appelé température de fusion, noté T_{f}) supérieur à 15°C. T_{f} est de façon préférée supérieur à 25°C, de façon plus préférée supérieur à 35°C, et de façon encore plus préférée supérieur à 50°C. Avantageusement, T_{f} peut être inférieur à 200°C, de plus préférée inférieur à 150°C, et de façon encore plus préférée inférieur à 130°C. A température ambiante, le composé oxydable selon l'invention se présente classiquement à l'état solide. A température ambiante, le contenu du container selon l'invention est à l'état solide. Il est entendu que le composé oxydable n'est pas en solution. Selon l'invention, la température ambiante est définie par une plage de température allant généralement de 10°C à 30°C, préférentiellement de 15°C à 30°C et plus préférentiellement de 15°C à 25°C. On n'exclut cependant pas les cas où le composé oxydable est soumis à des températures ambiantes plus extrêmes : la température ambiante peut par exemple atteindre dans certains endroits en hiver -15°C ou -20°C. La température ambiante peut être dans ce cas définie par une plage de température allant de -20°C à 10°C, préférentiellement de -15°C à 0°C.

Le composé oxydable selon l'invention peut être un composé pur ou il s'agit d'un mélange oxydable ayant un point de fusion supérieur à 15°C.

Le composé oxydable selon l'invention est un composé inhibiteur de polymérisation. Les inhibiteurs de polymérisation sont destinés à empêcher la polymérisation de monomères, par exemple la polymérisation radicalaire de monomères à insaturation éthylénique, pendant leur préparation industrielle, leur stockage et/ou leur transport. Le composé inhibiteur de polymérisation peut être un composé pur, un mélange de plusieurs composés eux-mêmes inhibiteurs de polymérisation, ou bien encore un composé essentiellement constitué d'un ou de plusieurs composés inhibiteurs de polymérisation et d'additifs, les additifs représentant de préférence moins de 10% en poids, de façon plus préférée moins de 5% en poids, et de façon encore plus préférée moins de 1% en poids de la composition totale. Parmi les composés inhibiteurs de polymérisation, on peut citer le p-méthoxyphénol, le 4-tert-butyl-catéchol, le pyrocatéchol, l'hydroquinone, le Topanol A (2,4-diméthyl,6-tertiobutyl-phénol), la phénothiazine, le dibutyl-dithio-carbamate de cuivre, la benzoquinone et leurs mélanges. Le composé oxydable selon l'invention peut être un composé essentiellement constitué d'un ou de plusieurs composés inhibiteurs de polymérisation choisis dans le groupe constitué par p-méthoxyphénol, l'hydroquinone et le 4-tert-butyl-catéchol. Tout particulièrement, le composé oxydable selon l'invention est essentiellement constitué de p-méthoxyphénol (noté PMP).

Classiquement, les composés inhibiteurs de polymérisation qui ont un point de fusion supérieur à 15°C tels que le PMP sont produits sous forme solide, par exemple, sous forme de poudre, d'écailles, de pellets, d'extrudés, de sphères, de billes ou de pastilles. Ils peuvent également se présenter sous forme liquide si on les chauffe au-delà de leur température de fusion. Pour leur transport et leur stockage, ces composés oxydables peuvent être conditionnés dans des containers, notamment lorsqu'ils sont à l'état liquide.

Dans la présente invention, le composé oxydable est contenu dans un container. Dans le présent exposé, le terme « container » inclut les fûts, les containers à déchargement par le bas et les citernes mobiles à déchargement par le haut. Ce container peut avoir n'importe quelle structure permettant de remplir sa fonction et être calorifugé ou non. De préférence, le container selon l'invention est un container de type GRV (grand récipient pour vrac), comme cela est bien connu dans le domaine technique. Différents types de containers sont disponibles dans le commerce, par exemple chez les fabricants GLI, SCHÄFER ou UCON.

Le volume du container selon l'invention peut être compris entre 50 L et 10000 L, de façon plus préférée entre 500 L et 2500 L. Selon un mode de réalisation de l'invention, le container est un petit container, dont le volume est compris de préférence entre 500 L et 2000 L.

Le conteneur peut être construit en acier revêtu mais préférentiellement en inox. Lorsque le container est adapté à un déchargement pas le bas, celui-ci présente de préférence un minimum d'obstacle à l'écoulement du produit à l'intérieur et un fond dont la forme spéciale favorise l'écoulement du produit fondu. Différentes technologies de vannes de fond sont possibles. On peut choisir préférentiellement une vanne de fond à papillon disposée au plus près du fond du conteneur pour réduire toute zone froide.

Un moyen de chauffage est utilisé dans le procédé selon l'invention. Ce moyen de chauffage peut être choisi parmi tous les dispositifs connus de l'homme du métier permettant de modifier la température à l'intérieur du container. Un seul ou plusieurs moyens de chauffage identiques ou différents peuvent être utilisés simultanément ou alternativement. Le ou les moyens de chauffage peuvent être des dispositifs intégrés dans le container et être indissociables de celui-ci, ou bien des dispositifs liés au container mais amovibles, ou bien encore des dispositifs indépendants du container.

Le conteneur peut être isolé thermiquement du milieu ambiant par un calorifuge qui lui est propre ou par tout dispositif dissocié du conteneur à même de remplir la fonction.

Selon un premier mode de réalisation, le container est équipé d'une paroi et préférentiellement d'un fond à double enveloppe dans laquelle un fluide caloporteur peut circuler. Le fluide caloporteur peut notamment être de l'eau liquide ou de la vapeur d'eau.

Selon un autre mode de réalisation, le container est muni de résistances chauffantes.

Selon encore un autre mode de réalisation, le moyen de chauffage consiste en une couverture chauffante ou une coquille chauffante qui peut être placée autour du container quand ce dernier n'est pas calorifugé.

Selon encore un autre mode de réalisation, le conteneur est muni d'un serpentin ou d'une épingle de chauffe dans lesquels un fluide caloporteur peut circuler

Selon encore un autre mode de réalisation, le moyen de chauffage consiste en une étuve ou une chambre chaude dans laquelle le container peut être placée.

L'invention concerne un procédé de déchargement dudit container contenant un composé oxydable ayant un point de fusion T_{f} supérieur à 15°C. Ce procédé comprend une première étape (a) qui consiste à chauffer le container à l'aide d'un moyen de chauffage jusqu'à ce que le composé oxydable soit liquide, la température du moyen de chauffage restant inférieure ou égale à T_{f} + 105°C, et de façon préférée T_{f} + 70°C.

Cette étape permet avantageusement de rendre le composé suffisamment liquide pour qu'il puisse être déchargé du container. Dans le présent exposé, on peut considérer qu'un composé est liquide lorsque sa viscosité est avantageusement inférieure ou égale à 100 Pa.s, de façon préférée 1 Pa.s, et de façon plus préférée 10 mPa.s, mesurée à l'aide d'un viscosimètre Rheomat 30 pour des vitesses de cisaillement de 100 s⁻¹ à 500 s⁻¹. La durée et la température de chauffage sont donc adaptées pour que ce résultat soit obtenu. La durée et la température de chauffage peuvent dépendre de la forme physique, de la quantité et de la température de départ du composé oxydable à chauffer, mais également de la présence d'un calorifuge, de la température ambiante et du moyen de chauffage utilisé.

De façon générale, la durée de l'étape (a) de chauffage peut être comprise entre 1 heure et 10 jours, de façon plus préférée entre 2 heures et 5 jours, et de façon encore plus préférée entre 4 heures et 48 heures. De préférence, lorsque la température est choisie plutôt dans le bas de la fourchette décrite, la durée est choisie plutôt dans le haut de la fourchette, et inversement.

Il peut cependant être difficile de maîtriser l'homogénéité du chauffage à l'intérieur du container. On peut considérer que la totalité du composé a atteint l'état liquide lorsque la température du composé, mesurée dans le container, est supérieure d'au moins 10°C, de façon plus préférée d'au moins 20°C à la température de fusion du composé T_{f}, et cela pendant une durée d'au moins 2 heures, de façon préférée d'au moins 5 heures, et de façon plus préférée d'au moins 8 heures. La température du composé oxydable à l'issue de l'étape (a) de chauffage est de façon préférée d'au plus T_{f} + 100°C, de façon plus préférée T_{f} + 65°C. La température du composé oxydable à l'intérieur du container peut être par exemple mesurée à l'aide d'une canne pyrométrique amovible ou non dans un doigt de gant, ou bien à l'aide de tout type de capteur de température avec ou sans contact disposé de façon permanente ou non sur le container. Cette condition sur la température du composé est de façon préférée satisfaite en au moins un point à l'intérieur du container, de façon plus préférée en au moins deux points à l'intérieur du container, et de façon encore plus préférée en au moins trois points à l'intérieur du container. Ces points peuvent être choisis dans la partie centrale du container, vers les parois ou proches de la vanne de sortie.

Les inventeurs ont toutefois constaté que le chauffage du composé oxydable pouvait avoir pour effet de dégrader le composé. Ils ont alors découvert que, pour éviter un échauffement local du composé oxydable, l'étape de chauffage devait être conduite de manière à ce que la température du moyen de chauffage reste en permanence inférieure ou égale à une certaine température. Selon la présente invention, durant l'étape de chauffage (a), la température du moyen de chauffage reste inférieur ou égal à T_{f} + 105°C, et de façon préférée T_{f} + 70°C, T_{f} étant la température de fusion du composé oxydable. De cette façon, la température des parois chauffées du container en contact avec le composé oxydable reste inférieur ou égal à T_{f} + 105°C, de façon préférée T_{f} + 70°C.

La mise en œuvrede l'étape (a) de chauffage dépend notamment du moyen de chauffage utilisé et de la nature des fluides ou mélange de fluides engagés. Le contrôle de la température du moyen de chauffage peut être exercé de manière connue par l'homme du métier en fonction du type de moyen de chauffage utilisé. Par exemple, lorsque le moyen de chauffage consiste entre un fluide caloporteur circulant dans une double enveloppe ou dans un serpentin, la température du moyen de chauffage peut être contrôlée via la température du fluide caloporteur. Lorsque le moyen de chauffe consiste en un dispositif électrique, la température du moyen de chauffage peut être contrôlée via l'alimentation électrique. Lorsque le moyen de chauffe consiste en une étuve ou une chambre chaude, la température du moyen de chauffage peut être contrôlée via un thermostat fixant la température de cette étuve ou chambre chaude. Selon un mode de réalisation, le container est muni d'une double enveloppe et l'étape (a) de chauffage consiste à faire circuler dans la double enveloppe du container de l'eau entre environ 75°C et environ 95°C ou de la vapeur d'eau détendue entre environ 100°C et environ 160°C pendant une durée déterminée en fonction de la température ambiante et de l'état de solidification du produit dans le conteneur.

Selon un mode de réalisation spécifique, le composé oxydable ayant un point de fusion T_{f} supérieur à 15°C est le p-méthoxyphénol et l'étape (a) consiste à chauffer le container à l'aide du moyen de chauffage jusqu'à ce que le composé oxydable soit liquide, la température du moyen de chauffage restant inférieure ou égal à 160°C, de façon préférée inférieure ou égal à 125°C. De préférence, la température du composé oxydable à la fin de l'étape de chauffage est comprise entre 65°C et 155°C, de façon plus préférée entre 75°C et 120°C.

Selon un mode de réalisation particulièrement avantageux, l'étape (a) est mise en œuvresous atmosphère inerte.

Dans l'exposé qui suit, l'expression « atmosphère inerte » désigne notamment tout gaz dont la teneur en oxygène est plus faible que celle de l'air. L'atmosphère inerte est constituée d'un gaz qui peut être choisi dans le groupe constitué par l'azote, l'argon, l'air appauvri, et le mélange de ceux-ci. L'expression « air appauvri » désigne de l'air contenant moins de 20% d'oxygène. De préférence, l'étape (a) est mise en œuvresous azote. De préférence, l'atmosphère inerte a une faible humidité, par exemple un taux d'humidité inférieur à 1000 ppm d'eau. De préférence, il s'agit d'azote industriel.

Pour que l'étape (a) soit mise en œuvresous atmosphère inerte, le container peut être préalablement inerté avant son remplissage et lors du chauffage, être relié par son évent à une arrivée d'un gaz inerte sous faible pression.

A l'issue de l'étape (a) de chauffage, le composé oxydable à l'état liquide est déchargé du container. Le déchargement peut être effectué selon toute technique appropriée, notamment en fonction de l'équipement du container. Le déchargement peut par exemple être effectué par le haut du container ou par le bas du container soit par vidange gravitaire, soit par vidange par pression, soit par pompage soit par système à éjecteur. Le déchargement peut être total ou partiel,

Lors du déchargement, le composé oxydable est transporté du container vers un autre récipient. Selon un mode de réalisation, il peut s'agir d'une cuve ou d'un réacteur contenant une composition de monomère ou de polymère. Selon un autre mode de réalisation, il peut s'agir d'un autre container, par exemple un container de plus petit volume ou de plus grand volume.

De préférence, le déchargement est effectué sous atmosphère inerte. Pour cela, le container peut être relié par son évent à une arrivée d'un gaz inerte sec. Selon un mode de réalisation, le container et l'autre récipient dans lequel le composé oxydable est déchargé constituent ensemble un système fermé. Ce mode de réalisation est avantageux lorsqu'on ne souhaite pas que le composé oxydable soit mis en contact avec une atmosphère non contrôlée, ou bien si l'autre récipient dans lequel le composé oxydable est déchargé contient par ailleurs un composé qu'on ne souhaite pas mettre en contact avec une atmosphère non contrôlée.

Les étapes (a) et (b) selon l'invention peuvent être mises en œuvredans le même lieu ou dans des lieux différents. En outre, ces étapes peuvent être enchaînées ou pas. Lorsque ces étapes ne sont pas enchainées, la durée entre la fin de l'étape (a) et le début de l'étape (b) peut être comprise entre 5 minutes et plusieurs jours, par exemple 2 jours. En effet, lorsque le composé oxydable a atteint l'état liquide à la fin de l'étape (a), il peut conserver cet état pendent une certaine durée, sans régulation de température. Cette durée peut dépendre notamment du type de container et de son isolation thermique. On veillera à ce que le composé soit toujours à l'état liquide lors de l'étape (b) de déchargement.

Les moyens de liaison liquide et gaz entre le conteneur et le récipient de déchargement sont de préférence les plus courts possible et de façon préférée calorifugés par tout moyen permettant la réduction des pertes thermiques.

Le procédé de déchargement tel que décrit ci-avant permet avantageusement de résoudre les problèmes techniques qui peuvent être rencontrés lors du déchargement à l'état liquide de matière oxydable solide à température ambiante. En particulier, cette procédure de déchargement permet d'éviter la coloration des composés inhibiteurs de polymérisation tels que le PMP.

Ce procédé de déchargement peut être associé à d'autres étapes de manutention telles que le chargement et le transport. C'est pourquoi la présente invention a également pour objet un procédé de manutention d'un composé oxydable ayant un point de fusion T_{f} supérieur à 15°C comprenant les étapes :
- de chargement d'un container avec un composé oxydable,
- de stockage et/ou de transport du container chargé,
- de déchargement du container,
- et éventuellement de retour du container après déchargement,
caractérisé en ce que l'étape de déchargement est mise en œuvreselon le procédé décrit ci-avant.

Le chargement du container avec le composé oxydable peut être effectué selon toute technique appropriée, notamment en fonction du type de container et de la forme physique du composé. Le composé oxydable peut être chargé dans le container sous forme liquide ou sous forme solide, de préférence sous forme liquide à une température supérieure à T_{f}. Ce procédé est avantageux dans la mesure où le composé n'a pas besoin d'être mis en forme en sortie de sa chaine de fabrication. Lors de l'étape de chargement, le container peut être chauffé ou froid.

Le chargement peut être réalisé sans précautions particulières. Cependant, un chargement en système fermé, c'est-à-dire sans que le composé oxydable soit en contact avec l'atmosphère ambiante, peut être préférable pour des raisons d'hygiène, de sécurité, et pour prévenir la dégradation du composé oxydable. Dans cette configuration, l'évent est de préférence réchauffé pour éviter tout risque de sublimation du produit.

Par ailleurs, le chargement peut être réalisé sous atmosphère inerte. Pour cela, un gaz inerte peut être injecté dans le container pendant et/ou juste après et de façon préférée avant le chargement du composé oxydable. Avant le chargement, le container peut être rempli d'un gaz inerte. Pendant le chargement, l'atmosphère inerte peut être maintenue grâce par exemple à un balayage de gaz inerte. Enfin, juste après le chargement, un ciel de gaz inerte peut être créé ou maintenu.

Selon un mode de réalisation préféré, le procédé de chargement d'un container avec un composé oxydable ayant un point de fusion T_{f} supérieur à 15°C comprend les étapes consistant à :
- placer le container sous atmosphère inerte ;
- charger ledit composé oxydable à l'état liquide dans le container.

Ce procédé de chargement peut être un objet de la présente invention. Selon un mode de réalisation préféré, le composé oxydable est chargé dans le container à l'état liquide sous atmosphère inerte.

Le composé oxydable une fois chargé dans le container peut être stocké et/ou transporté. De préférence, la température du container n'est pas régulée pendant l'étape de stockage et/ou de transport, ce qui permet de diminuer les frais. Dans des conditions normales, si le container n'est pas maintenu à une température supérieure à la température de fusion T_{f} du composé oxydable, le composé va se solidifier à l'intérieur du container. La durée nécessaire pour que le composé soit à l'état solide dans le container peut varier en fonction de la nature du composé, du type de container, de la température ambiante et des conditions de stockage et/ou de transport. De façon très générale, le composé peut mettre de 2 à 8 ou 10 jours pour prendre une forme solide.

Durant le stockage et le transport, tous les orifices du conteneur demeurent préférentiellement hermétiquement fermés.

Arrivé en son point de déchargement l'homme du métier peut mettre en œuvrela procédure de déchargement selon la présente invention pour décharger facilement et efficacement, à l'état liquide, le composé oxydable, sans dégrader le composé.

Après le déchargement total ou partiel du container, celui-ci peut être transporté jusqu'à son point de départ pour être éventuellement réutilisé. Selon un mode de réalisation préféré, le container après déchargement est mis ou maintenu sous atmosphère inerte, puis retourné sous atmosphère inerte. Cela est particulièrement avantageux si du composé oxydable se trouve toujours dans le container. Cela est aussi avantageux si le container est ensuite réutilisé, l'étape initiale d'inertage du container étant alors déjà effectuée.

Un dispositif permettant de mettre en œuvrele procédé de déchargement décrit ci-dessus, ainsi que le procédé de manutention décrit ci-dessus comprend :
(A) un container, éventuellement muni d'un moyen de chauffage, utilisable dans le procédé selon l'invention, et
(B) des instructions pour procéder au déchargement dudit container conformément audit procédé.

Le container (A) peut être tel que décrit de préférence ci-dessus. En particulier, le container peut être muni de préférence d'au moins un moyen de chauffage, et que ledit moyen de chauffage est choisi parmi un dispositif intégré et indissociable du container, un dispositif lié au container mais amovible, ou bien un dispositif indépendant du container. Les instructions (B) décrivent les étapes devant être mises en œuvrepour procéder au déchargement du container (A) selon l'invention. En outre, ces instructions peuvent décrire des modes préférés de réalisation, ainsi que d'autres étapes de manutention telles que le chargement, le stockage, le transport et le retour du container. De préférence, ces instructions se présentent sous forme écrite sur un support papier, mais il est également possible qu'elles soient enregistrées sur un support électronique, sous forme écrite, audio ou vidéo.

L'invention sera expliquée plus en détail au moyen des exemples ci-après donnés à titre illustratif et non limitatif.

### EXEMPLES :

### Exemple de chauffage à l'eau chaude :

Un containeur en inox de 1000 kg a été rempli avec du PMP fondu et le conteneur a été isolé. Lorsque la température du produit dans le container a atteint 28°C, le PMP se trouvait alors à l'état solide dans le container. Le container a été mis sous atmosphère inerte. On a chauffé le container en faisant circuler dans la double enveloppe du container de l'eau chaude à une température de 70 °C pendant une durée de 4 jours sous azote. A l'issue de ce chauffage, le produit obtenu était fluide et homogène et le container a été vidangé par gravité dans un container de stockage. Le produit présentait une coloration inférieure à 100 Hazen.

Ce mode opératoire a été reproduit, mais sans inertage du container. Le container a alors été chauffé à l'eau chaude à 70°C sous air pendant une durée de 4 jours. Le produit obtenu était fluide présentait, après vidange, une coloration de 220 Hazen.

Par comparaison, on a reproduit ce mode opératoire, mais l'étape de chauffage n'a duré que 20 heures. Le produit obtenu était fluide mais pas homogène et présentait des agglomérats de PMP solide. Après vidange, le container a été pesé et il y avait 130 kg de PMP solide à l'intérieur du container.

### Exemple de chauffage à la vapeur d'eau à 140°C :

Un containeur en inox de 1000 kg a été rempli avec du PMP fondu et le conteneur a été isolé. Lorsque la température du produit dans le container a atteint 28°C, le PMP se trouvait alors à l'état solide dans le container. Le container a été mis sous atmosphère inerte. On a chauffé le container avec de la vapeur d'eau à 140°C sous azote pendant 24 heures. Le produit obtenu était fluide et homogène. Le container a été vidangé par gravité. Le produit présentait une coloration égale à 100 Hazen.

Ce mode opératoire a été reproduit, mais sans inertage du container. Le container a été chauffé à la vapeur d'eau à 140°C sous air pendant 24 heures. Le produit obtenu était fluide et présentait, après vidange, une coloration égale à 150 Hazen.

Par comparaison, on a reproduit ce mode opératoire, mais en chauffant le container à la vapeur 6 bars avec une température de paroi de 165°C sous air, pendant 24 heures. Le produit obtenu était fluide et, après vidange, il présentait une coloration supérieure à 300 Hazen.

### Exemple de chauffage à la vapeur détendue à 100°C :

Un containeur en inox de 1000 kg a été rempli avec du PMP fondu et le conteneur a été isolé. Lorsque la température du produit dans le container a atteint 28°C, le PMP se trouvait alors à l'état solide dans le container. Le container a été mis sous atmosphère inerte. Le container a été chauffé à l'aide de vapeur d'eau détendue à 100 °C sous azote pendant 24 heures. Le produit obtenu était fluide et homogène et le container a été vidangé par gravité. Le produit présentait une coloration égale à 60 Hazen.

## Revendications

1. Procédé de déchargement d'un container contenant un composé oxydable ayant un point de fusion Tf supérieur à 15°C, comprenant les étapes consistant à :
a) chauffer le container à l'aide d'un moyen de chauffage jusqu'à ce que le composé oxydable soit liquide, la température du moyen de chauffage restant inférieure ou égale à T_{f} + 105°C ;
b) décharger le composé oxydable à l'état liquide,
**caractérisé en ce que** le composé oxydable ayant un point de fusion Tf supérieur à 15°C est un composé inhibiteur de polymérisation.

2. Procédé selon la revendication 1, **caractérisé en ce que** l'étape (a) est mise en œuvresous atmosphère inerte, de préférence avec une faible humidité.

3. Procédé selon la revendication 2, **caractérisé en ce que** le composé oxydable ayant un point de fusion Tf supérieur à 15°C est un composé essentiellement constitué d'un ou de plusieurs composés inhibiteurs de polymérisation choisis dans le groupe constitué par p-méthoxyphénol et le 4-tert-butyl-catéchol.

4. Procédé selon la revendication 3, **caractérisé en ce que** le composé oxydable ayant un point de fusion T_{f} supérieur à 15°C est un composé essentiellement constitué de p-méthoxyphénol.

5. Procédé selon l'une quelconque des revendications 1 à 4, **caractérisé en ce que** la totalité du composé a atteint l'état liquide lorsque la température du composé, mesurée au milieu du container, est supérieure d'au moins 10°C, de façon plus préférée d'au moins 20°C à la température de fusion du composé Tf, et cela pendant une durée d'au moins 2 heures, de façon préférée d'au moins 5 heures, et de façon plus préférée d'au moins 8 heures.

6. Procédé selon l'une quelconque des revendications 1 à 5, **caractérisé en ce que** le container est muni d'une double enveloppe et l'étape (a) de chauffage consiste à faire circuler dans la double enveloppe du container de l'eau entre environ 75°C et environ 95°C.

7. Procédé selon l'une quelconque des revendications 1 à 5, **caractérisé en ce que** le container est muni d'une double enveloppe et l'étape (a) de chauffage consiste à faire circuler dans la double enveloppe du container de la vapeur d'eau détendue entre environ 100°C et environ 160°C.

8. Procédé selon l'une quelconque des revendications 1 à 7, **caractérisé en ce que** le composé oxydable ayant un point de fusion Tf supérieur à 15°C est le p-méthoxyphénol et l'étape (a) consiste à chauffer le container à l'aide du moyen de chauffage jusqu'à ce que le composé oxydable soit liquide, la température du moyen de chauffage restant inférieure ou égal à 160°C, la température du composé oxydable à la fin de l'étape de chauffage étant comprise entre 65°C et 155°C.

9. Procédé selon la revendications 8, **caractérisé en ce que** le composé oxydable ayant un point de fusion Tf supérieur à 15°C est le p-méthoxyphénol et l'étape (a) consiste à chauffer le container à l'aide du moyen de chauffage jusqu'à ce que le composé oxydable soit liquide, la température du moyen de chauffage restant inférieure ou égal à 125°C, la température du composé oxydable à la fin de l'étape de chauffage étant comprise entre 75°C et 120°C.

10. Procédé de manutention d'un composé oxydable ayant un point de fusion T_{f} supérieur à 15°C comprenant les étapes de chargement d'un container avec un composé oxydable, de stockage et/ou de transport du container chargé, de déchargement du container, et éventuellement de retour du container après déchargement, **caractérisé en ce que** l'étape de déchargement est mise en œuvre selon l'une quelconque des revendications 1 à 9.

11. Procédé selon la revendication 10, **caractérisé en ce que** lors de l'étape de chargement, le composé oxydable est chargé dans le container à l'état liquide sous atmosphère inerte.

12. Procédé selon la revendication 10 ou la revendication 11, **caractérisé en ce que** la température du container n'est pas régulée pendant l'étape de stockage et/ou de transport.

## Patentansprüche

1. Verfahren zum Entleeren eines Containers, der eine oxidierbare Verbindung mit einem Schmelzpunkt T_{f} über 15 °C enthält, umfassend die folgenden Schritte:
a) Erhitzen des Containers mithilfe einer Heizeinrichtung, bis die oxidierbare Verbindung flüssig ist, wobei die Temperatur der Heizeinrichtung kleiner oder gleich T_{f} + 105 °C bleibt;
b) Entleeren der oxidierbaren Verbindung in flüssigem Zustand,
**dadurch gekennzeichnet, dass** die oxidierbare Verbindung mit einem Schmelzpunkt T_{f} über 15 °C eine polymerisationsinhibierende Verbindung ist.

2. Verfahren nach Anspruch 1, **dadurch gekennzeichnet, dass** Schritt (a) unter Inertatmosphäre durchgeführt wird, bevorzugt bei geringer Feuchte.

3. Verfahren nach Anspruch 2, **dadurch gekennzeichnet, dass** die oxidierbare Verbindung mit einem Schmelzpunkt T_{f} über 15 °C eine Verbindung ist, die im Wesentlichen aus einer oder mehreren polymerisationsinhibierenden Verbindungen besteht, die aus der aus p-Methoxyphenol und 4-tert-Butylcatechol bestehenden Gruppe gewählt sind.

4. Verfahren nach Anspruch 3, **dadurch gekennzeichnet, dass** die oxidierbare Verbindung mit einem Schmelzpunkt T_{f} über 15 °C eine Verbindung ist, die im Wesentlichen aus p-Methoxyphenol besteht.

5. Verfahren nach einem der Ansprüche 1 bis 4, **dadurch gekennzeichnet, dass** die Gesamtheit der Verbindung den flüssigen Zustand erreicht hat, wenn die Temperatur der Verbindung, gemessen in der Mitte des Containers, um mindestens 10 °C, noch bevorzugter um mindestens 20 °C über der Schmelztemperatur der Verbindung T_{f} liegt, und dies während einer Dauer von mindestens 2 Stunden, bevorzugt mindestens 5 Stunden und noch bevorzugter mindestens 8 Stunden.

6. Verfahren nach einem der Ansprüche 1 bis 5, **dadurch gekennzeichnet, dass** der Container mit einem Doppelmantel versehen ist und der Schritt (a) des Erhitzens darin besteht, in dem Doppelmantel des Containers Wasser zwischen ungefähr 75 °C und ungefähr 95 °C zirkulieren zu lassen.

7. Verfahren nach einem der Ansprüche 1 bis 5, **dadurch gekennzeichnet, dass** der Container mit einem Doppelmantel versehen ist und der Schritt (a) des Erhitzens darin besteht, in dem Doppelmantel des Containers entspannten Wasserdampf zwischen ungefähr 100 °C und ungefähr 160 °C zirkulieren zu lassen.

8. Verfahren nach einem der Ansprüche 1 bis 7, **dadurch gekennzeichnet, dass** die oxidierbare Verbindung mit einem Schmelzpunkt T_{f} über 15 °C p-Methoxyphenol ist und der Schritt (a) darin besteht, den Container mithilfe der Heizeinrichtung zu erhitzen, bis die oxidierbare Verbindung flüssig ist, wobei die Temperatur der Heizeinrichtung kleiner oder gleich 160 °C bleibt, wobei die Temperatur der oxidierbaren Verbindung am Ende des Erhitzungsschritts zwischen 65 °C und 155 °C beträgt.

9. Verfahren nach Anspruch 8, **dadurch gekennzeichnet, dass** die oxidierbare Verbindung mit einem Schmelzpunkt T_{f} über 15 °C p-Methoxyphenol ist und der Schritt (a) darin besteht, den Container mithilfe der Heizeinrichtung zu erhitzen, bis die oxidierbare Verbindung flüssig ist, wobei die Temperatur der Heizeinrichtung kleiner oder gleich 125 °C bleibt, wobei die Temperatur der oxidierbaren Verbindung am Ende des Erhitzungsschritts zwischen 75 °C und 120 °C beträgt.

10. Verfahren zur Handhabung einer oxidierbaren Verbindung mit einem Schmelzpunkt T_{f} über 15 °C, umfassend die Schritte des Befüllens eines Containers mit einer oxidierbaren Verbindung, des Lagerns und/oder des Transportierens des befüllten Containers, des Entleerens des Containers und gegebenenfalls des Rückführens des Containers nach dem Entleeren, **dadurch gekennzeichnet, dass** der Schritt des Entleerens nach einem der Ansprüche 1 bis 9 durchgeführt wird.

11. Verfahren nach Anspruch 10, **dadurch gekennzeichnet, dass** beim Schritt des Befüllens die oxidierbare Verbindung im flüssigen Zustand unter Inertatmosphäre in den Container gefüllt wird.

12. Verfahren nach Anspruch 10 oder Anspruch 11, **dadurch gekennzeichnet, dass** die Temperatur des Containers während des Lagerungs- und/oder Transportschritts nicht geregelt wird.

## Claims

1. Process for unloading a container containing an oxidizable compound having a melting point Tₘ of greater than 15°C, comprising the steps consisting in:
a) heating the container using a heating means until the oxidizable compound is liquid, the temperature of the heating means remaining less than or equal to Tₘ + 105°C;
b) unloading the oxidizable compound in the liquid state,
**characterized in that** the oxidizable compound having a melting point Tₘ of greater than 15°C is a polymerization-inhibiting compound.

2. Process according to Claim 1, **characterized in that** step (a) is carried out under an inert atmosphere, preferably with a low moisture content.

3. Process according to Claim 2, **characterized in that** the oxidizable compound having a melting point Tₘ of greater than 15°C is a compound which is essentially composed of one or more polymerization-inhibiting compounds selected from the group consisting of p-methoxyphenol and 4-tert-butylcatechol.

4. Process according to Claim 3, **characterized in that** the oxidizable compound having a melting point Tₘ of greater than 15°C is a compound essentially composed of p-methoxyphenol.

5. Process according to any one of Claims 1 to 4, **characterized in that** all of the compound has reached the liquid state when the temperature of the compound, measured in the middle of the container, is greater than the melting point Tₘ of the compound by at least 10°C, more preferably by at least 20°C, for a duration of at least 2 hours, preferably at least 5 hours, and more preferably at least 8 hours.

6. Process according to any one of Claims 1 to 5, **characterized in that** the container is provided with a jacket and step (a) of heating consists in causing water between approximately 75°C and approximately 95°C to circulate in the jacket of the container.

7. Process according to any one of Claims 1 to 5, **characterized in that** the container is provided with a jacket and step (a) of heating consists in causing expanded steam between approximately 100°C and approximately 160°C to circulate in the jacket of the container.

8. Process according to any one of Claims 1 to 7, **characterized in that** the oxidizable compound having a melting point Tₘ of greater than 15°C is p-methoxyphenol and step (a) consists in heating the container using the heating means until the oxidizable compound is liquid, the temperature of the heating means remaining less than or equal to 160°C, the temperature of the oxidizable compound at the end of the heating step being between 65°C and 155°C.

9. Process according to Claim 8, **characterized in that** the oxidizable compound having a melting point Tₘ of greater than 15°C is p-methoxyphenol and step (a) consists in heating the container using the heating means until the oxidizable compound is liquid, the temperature of the heating means remaining less than or equal to 125°C, the temperature of the oxidizable compound at the end of the heating step being between 75°C and 120°C.

10. Process for handling an oxidizable compound having a melting point Tₘ of greater than 15°C, comprising the steps of loading a container with an oxidizable compound, of storage and/or transport of the loaded container, of unloading the container and optionally of returning the container after unloading, **characterized in that** the unloading step is carried out according to any one of Claims 1 to 9.

11. Process according to Claim 10, **characterized in that**, during the loading step, the oxidizable compound is loaded into the container in the liquid state under an inert atmosphere.

12. Process according to Claim 10 or Claim 11, **characterized in that** the temperature of the container is not regulated during the step of storage and/or transport.
